# EUROPEAN PATENT APPLICATION

(11) **EP 2 319 849 A1**
(43) Date of publication of application: **11.05.2011**
(21) Application number: 09800427.8
(22) Date of filing: 23.07.2009
(51) Int. Cl.: C07F 5/05, A61K 51/00, A61P 35/00

(54) **METHOD FOR PRODUCING BSH DERIVATIVE AND BSH DERIVATIVE**

(30) Priority: 24.07.2008 JP 2008191279
(71) Applicant: Stella Pharma Corporation, Osaka-shi, Osaka 541-0043 (JP)
(72) Inventor: KIRIHATA,Mitsunori, Sakai-shi Osaka 599-8231 (JP); ASANO,Tomoyuki, Osaka-shi Osaka 541-0043 (JP); UEHARA,Kohki, Osaka-shi Osaka 541-0043 (JP); HATTORI,Yoshihide, Sakai-shi Osaka 599-8231 (JP)
(74) Representative: Hart-Davis, Jason
(86) International application number: PCT/JP2009/063160
(87) International publication number: WO 2010/010913

(57) **Abstract**

Disclosed is a method for producing a BSH derivative, which comprises a step of addition-reacting BSH with an α,β-unsaturated nitrile compound in the presence of a base. Various BSH derivatives obtained by the method are also disclosed.

## Description

### Technical Field

The present invention relates to a method for producing a mercaptoundecahydrododecaborate (BSH: borocaptate) derivative, and a novel BSH derivative. More specifically, the present invention relates to a method for producing a BSH derivative, which is useful as a therapeutic agent for neutron capture used in boron neutron capture therapy (BNCT), by modifying thiol groups of BSH, and a novel BSH derivative.

### Background Art

Recently, attention has been drawn to boron neutron capture therapy (BNCT) as a novel cancer therapeutic method utilizing a radioisotope. The boron neutron capture therapy is a therapeutic method in which a boron compound containing boron-10 isotope (10B) is incorporated into cancer cells and the cancer cells are irradiated with low energy neutron (for example, thermal neutron), and thus the cancer cells are locally destroyed by a nuclear reaction which arises in the cells. In this therapeutic method, since it is important to selectively accumulate the boron compound containing 10B in cells of cancerous tissues so as to enhance the therapeutic effect, a boron compound which is selectively incorporated into cancer cells has been developed.

There have been synthesized so far, as a drug used in BNCT, some boron-containing compounds in which boron atoms or boron atomic groups are introduced as a basic skelton. Examples of the drug used in actual clinical practice include p-boronophenylalanine (BPA) and BSH. Among these drugs, BSH is mainly used for the treatment of brain tumor in the form of a sodium salt, and utility thereof has been confirmed (see, for example, Non-Patent Documents I to 8).

On the other hand, it is a very important objective to develop a boron compound which is incorporated into cancerous tissues more quickly and more specifically. However, there have been scarcely reported examples concerning derivatives of BSH, heretofore.

### Prior Art Documents

### Non-Patent Documents

Non-Patent Document 1: I. M. Wyzlic et al., Tetrahedron Lett., 1992, 33, 7489-7490;
Non-Patent Document 2: W. Tjark, J. Organomet. Chem., 2000, 614-615, 37-47;
Non-Patent Document 3: K. Imamura et al., Bull. Chem. Soc. Jpn., 1997, 70. 3103-3110;
Non-Patent Document 4 : A. S. Al-Madhorn et al., J. Med. Chem., 2002, 45, 4018-4028;
Non-Patent Document 5 : F. Compostella et al., Res. Develop. Neutron Capture Ther., 2002, 81-84;
Non-Patent Document 6 : S. B kahl et al., Progress in Neutron Capture Therapy for Cancer, Plenum Press, New York 1992, 223;
Non-Patent Document 7 : J. Cai et al., J. Med. Chem., 1997, 40, 3887-3896;
Non-Patent Document 8 : H.Lim et al., Res. Develop. Neutron Capture Ther., 2002, 37-42

### Summary of the Invention

### Problems to be Solved by the Invention

It is desired to develop, as a boron compound incorporated into cancer cells which can be utilized for BNCT, a compound which enables quick and accurate accumulation of BSH to the affected part.

Thus, an objective of the present invention is to provide a method for synthesizing a derivative in which thiol groups of BSH are modified, quickly and simply, and a BSH derivative obtained by the same.

### Means for Solving the Problems

The present inventors have intensively studied and, as a result, have found that the above objective can be achieved by modifying SH groups of BSH through paying attention to a feature of its behavior as described hereinafter, and thus the present invention has been completed.

That is, the present invention relates to a method for producing a BSH derivative, which comprises the step of an addition reaction of BSH with an α,β-unsaturated carbonyl compound or an α,β-unsaturated nitrile compound in the presence of a base.

In the method for producing a BSH derivative, the α,β-unsaturated carbonyl compound or the α,β-unsaturated nitrile compound can be one kind selected from the group consisting of methyl N^{α}-(p-cyanobenzoyl)-2-amino acrylate, acrolein, crotonaldehyde, cinnamaldehyde, mesityl oxide, benzalacetone, dibenzalacetone, benzalacetophenone, dypnone, acrylic acid, crotonic acid, isocrotonic acid, methacrylic acid, sorbic acid, cinnamic acid, maleic acid, fumaric acid, maleic anhydride, acrylonitrile, 2-butenedinitrile, methylene butyrolactone, oxofuran, methyl acrylate, acrylamide, methylene pentanedinitrile, methyl methacrylate, ethyl cinnamate, acrylonitrile, methyl vinyl ketone, cyclopentenone, methylcyclopentenone, cyclohexenone, propenoyloxazolidinone, ethyl propionate, methyl propionate, 3-methyl-3-buten-2-one, 1-penten-3-one and 2-methylenequinuclidin-3-one.

The present invention also relates to a BSH derivative represented by the general formula: 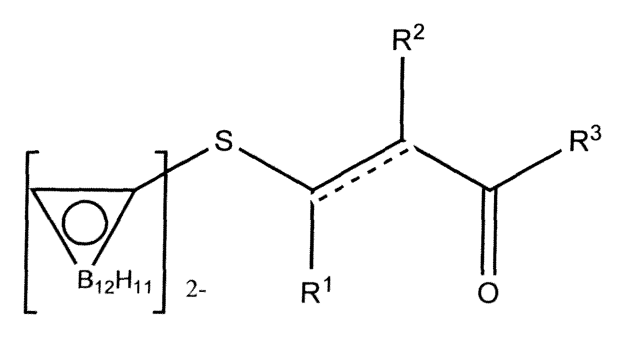 wherein a dashed line represents a bond or no bond; either R¹ or R² necessarily represents hydrogen, and both R¹ and R² may be hydrogens or either R¹ or R² may be an alkyl group having 1 to 6 carbon atoms, or a substituted amino group (in which a substituent represents a C1-6 alkyl group or a cyanobenzoyl group); and R³ represents hydrogen, an alkyl group having 1 to 6 carbon atoms, an alkoxyl group having 1 to 6 carbon atoms (provided that, when R³ is a C1 alkoxyl group and a dashed line is no bond, R² is not hydrogen) or NR⁴R⁵ (in which R⁴ and R⁵ independently represent hydrogen, an alkyl group having 1 to 6 carbon atoms, or NR⁴R⁵s are combined together to form a 4- to 10-membered, saturated or unsaturated ring having a hetero atom); or when R¹ is hydrogen, R² and R³ represent, together with adjacent carbonyl groups, an oxo-substituted C4-10 cycloalkane, an oxo-substituted unsaturated hydrocarbon ring group having 4 to 10 carbon atoms, a 4- to 10-membered oxo-substituted saturated or unsaturated heterocyclic group having at least one oxygen or nitrogen or sulfur atom, an oxo-substituted C6-12 crosslinked cyclic hydrocarbon group, or a 6- to 12-membered oxo-substituted crosslinked ring group having at least one oxygen or nitrogen or sulfur atom).

The present invention also relates to a BSH derivative represented by the general formula: 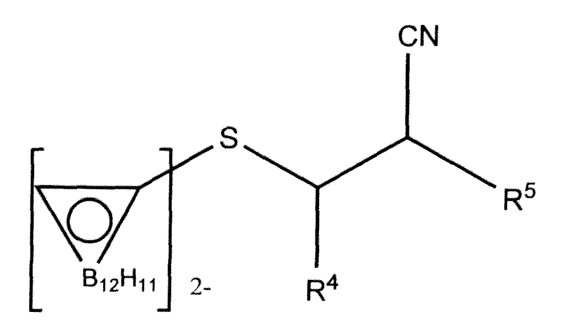 wherein either R⁴ or R⁵ necessarily represents hydrogen, but not both R⁴ and R⁵ are hydrogens, and R⁴ or R⁵ represents an alkyl group having 1 to 6 carbon atoms, a cyano C1-6 alkyl group or cyano, or R⁴ and R⁵ represents, together with adjacent cyano groups, a cyano group-substituted C4-10 cycloalkane, a cyano-substituted unsaturated hydrocarbon ring group having 4 to 10 carbon atoms, a 4- to 10-membered cyano-substituted saturated or unsaturated heterocyclic group having at least one oxygen or nitrogen or sulfur atom, a cyano-substituted C6-12 crosslinked cyclic hydrocarbon group, or a 6- to 12-membered cyano-substituted crosslinked ring group having at least one oxygen or nitrogen or sulfur atom).

The present invention also relates to a BSH derivative which is one kind selected from the group consisting of N^{α}- (p-cyanobenzoyl)-DL-3-(undecahydrododeca (¹⁰B) boranethio)-alanine methyl ester, 3-[undecahydrododeca (¹⁰B) boranethiomethyl] -dihydrofuran-2-one, 2-[undecahydrododeca (¹⁰B)boranethiomethyl]pentanedinitrile, 4-[undecahydrododeca(¹⁰B)boranethio]-dihydrofuran-2-one, methyl 3-[undecahydrododeca(¹⁰B)boranethio]-propionate, 2-[undecahydrododeca(¹⁰B)boranethio]-succinonitrile, 3-[undecahydrododeca(¹⁰B)boranethio]propionamide, 4-[undecahydrododeca(¹⁰B) boranethio]-butan-2-one, 3-[undecahydrododeca(¹⁰B)boranethio]-cyclopentanone, 3-methyl-4-[undecahydrododeca(¹⁰B)boranethio]-butan-2-one, 1-[undecahydrododeca(¹⁰B)boranethio]-pentan-3-one, 3-[undecahydrododeca(¹⁰B)boranethio]-cyclohexanone, ethyl E/Z-3-(undecahydrododeca(¹⁰B)boranethio)-acrylate, 3-(3-undecahydrododeca(¹⁰B)boranethio-propynoyl)-2-oxazolidinone and 2-[undecahydrododeca(¹⁰B)boranethiomethyl]-1-aza-bicyclo-[2.2.2]octan-3-one, or a pharmaceutically acceptable salt thereof.

### Effects of the Invention

It is possible to obtain a novel BSH derivative extremely simply and quickly by applying the production method of the present invention. Furthermore, it becomes possible to produce a BSH derivative using widely various compounds. The BSH derivative thus obtained is useful as BNCT which targets cancer cells.

### Mode for Carrying Out the Invention

Embodiments of the present invention will be described below.

BSH is a compound having a boron cluster structure of an icosahedron including boron, hydrogen and sulfur atoms. BSH has a so-called three center bond structure in which, regardless of an inorganic low molecular compound, the volume is larger than that of the benzene ring and three boron atoms have two electrons in common, and also has a specific structure in which electrons are localized.
In the present description, it may be conveniently represented by the following formula: 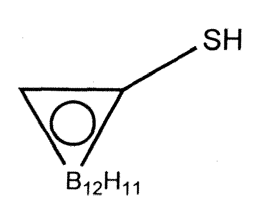 wherein all borons are 10B.

In the method for producing a BSH derivative of the present invention, the step of an addition reaction of BSH with an α,β-unsaturated carbonyl compound or an α,β-unsaturated nitrile compound in the presence of a base is included.

The α,β-unsaturated carbonyl compound has both a carbon-carbon double bond and a carbon-oxygen double bond, and includes all compounds in which these two bonds are mutually separated by only one carbon-carbon bond. Also, the α,β-unsaturated nitrile compound has both a carbon-carbon double bond and a carbon-nitrogen triple bond, and includes all compounds in which these two bonds are mutually separated by only one carbon-carbon bond.

Examples of a compound which can be particularly suitably used as the α,β-unsaturated carbonyl compound in the present invention include, but are not limited to, methyl N^{α}-(p-cyanobenzoyl)-2-amino acrylate, acrolein, crotonaldehyde, cinnamaldehyde, mesityl oxide, benzalacetone, dibenzalacetone, benzalacetophenone, dypnone, acrylic acid, crotonic acid, isocrotonic acid, methacrylic acid, sorbic acid, cinnamic acid, maleic acid, fumaric acid, maleic anhydride, methylene butyrolactone, oxofuran, methyl acrylate, acrylamide, methyl methacrylate, ethyl cinnamate, methyl vinyl ketone, cyclopentenone, methylcyclopentenone, cyclohexenone, 3-methyl-3-buten-2-one, 1-penten-3-one, propenoyloxazolidinone, ethyl propionate, methyl propionate and 2-methylenequinuclidin-3-one.

Examples of a compound which can be particularly suitably used as the α,β-unsaturated nitrile compound in the present invention include, but are not limited to, acrylonitrile, 2-butenedinitrile, acrylonitrile and methylene pentanedinitrile.

The reaction of the BSH of the present invention with the α,β-unsaturated carbonyl compound or the α,β-unsaturated nitrile compound is a reaction in which carboanions generated from active methylene are added to the conjugated end of a conjugated olefin in the presence of a basic catalyst.

The reaction can be carried out using mainly, as a medium, an alcohol solvent such as methanol or ethanol, or an ether-based solvent such as diethylether or dioxane, or a polar organic solvent such as benzene.

Examples of the basic catalyst include, but are not limited to, sodium ethoxide, dimethylaminopyridine and the like.

There is no particular limitation on the reaction temperature and the reaction time. However, the reaction can be performed at 0 to 50°C for 5 minutes to 3 hours.

Each product obtained in such a reaction may be isolated and purified, or may be subjected to the subsequent modification or reduction treatment as it is. The isolation and purification means include washing, extraction, recrystallization methods, various chromatographies and the like. In each product in each step, these isolation and purification means can also be used alone, or in appropriate combination of two or more kinds of them.

Particularly in the case of a reaction of BSH with an α,β-unsaturated carbonyl compound, the compound obtained by the reaction of the present invention can be represented by the following general formula: 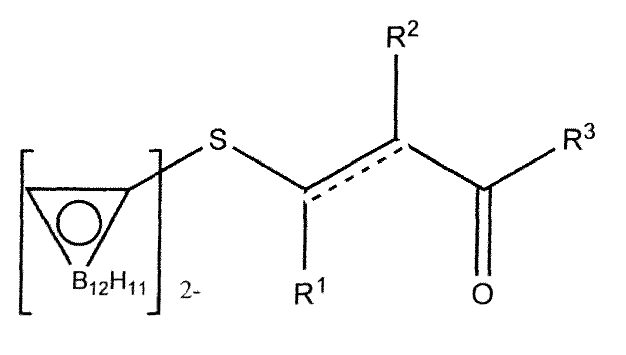 wherein a dashed line represents a bond or no bond; either R¹ or R² necessarily represents hydrogen, and both R¹ and R² may be hydrogens or either R¹ or R² may be an alkyl group having 1 to 6 carbon atoms, or a substituted amino group (in which a substituent represents a C1-6 alkyl group or a cyanobenzoyl group); and R³ represents hydrogen, an alkyl group having 1 to 6 carbon atoms, an alkoxyl group having 1 to 6 carbon atoms (provided that, when R³ is a C1 alkoxyl group, and a dashed line is no bond, R² is not hydrogen) or NR⁴R⁵ (in which R⁴ and R⁵ independently represent hydrogen, an alkyl group having 1 to 6 carbon atoms, or NR⁴R⁵s are combined together to form a 4- to 10-membered, saturated or unsaturated ring having a hetero atom); or when R¹ is hydrogen, R² and R³ represent, together with adjacent carbonyl groups, an oxo-substituted C4-10 cycloalkane, an oxo-substituted unsaturated hydrocarbon ring group having 4 to 10 carbon atoms, a 4- to 10-membered oxo-substituted saturated or unsaturated heterocyclic group having at least one oxygen or nitrogen or sulfur atom, an oxo-substituted C6-12 crosslinked cyclic hydrocarbon group, or a 6- to 12-membered oxo-substituted crosslinked ring group having at least one oxygen or nitrogen or sulfur atom).

Alternatively, in the case of a reaction of BSH with an α,β-unsaturated nitrile compound, it is possible to obtain a compound represented by the following formula: 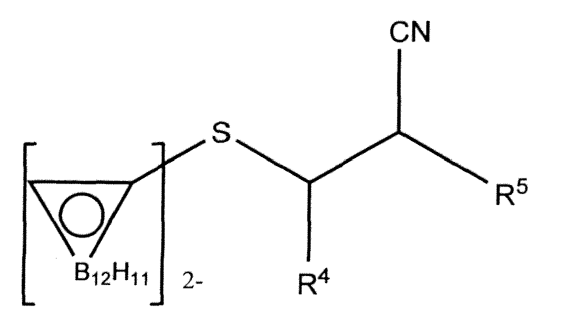 wherein either R⁴ or R⁵ necessarily represents hydrogen, but not both R⁴ and R⁵ are hydrogens, and either R⁴ or R⁵ represents an alkyl group having 1 to 6 carbon atoms, a cyano C1-6 alkyl group or cyano, or R⁴ and R⁵ represent, together with adjacent cyano groups, a cyano group-substituted C4-10 cycloalkane, a cyano-substituted unsaturated hydrocarbon ring group having 4 to 10 carbon atoms, a 4- to 10-membered cyano-substituted saturated or unsaturated heterocyclic group having at least one oxygen or nitrogen or sulfur atom, a cyano-substituted C6-12 crosslinked cyclic hydrocarbon group, or a 6- to 12-membered cyano-substituted crosslinked ring group having at least one oxygen or nitrogen or sulfur atom).

Preferred examples of the compound include, but are not particularly limited to, the following compounds: N^{α}-(p-cyanobenzoyl) -DL-3- (undecahydrododeca (¹⁰B) boranethio)-alanine methyl ester, 3-[undecahydrododeca (¹⁰B)boranethiomethyl]-dihydrofuran-2-one, 2-[undecahydrododeca(¹⁰B)boranethiomethyl]pentanedinitrile, 4-[undecahydrododeca(¹⁰B)boranethio]-dihydrofuran-2-one, methyl 3-[undecahydrododeca(¹⁰B)boranethio]-propionate, 2-[undecahydrododeca(¹⁰B)boranethio]-succinonitrile, 3-[undecahydrododeca(¹⁰B)boranethio]propionamide, 4-[undecahydrododeca(¹⁰B)boranethio]-butan-2-one, 3-[undecahydrododeca(¹⁰B)boranethio]-cyclopentanone, 3-methyl-4-[undecahydrododeca(¹⁰B)boranethio]-butan-2-one, 1-[undecahydrododeca(¹⁰B)boranethio]-pentan-3-one, 3-[undecahydrododeca(¹⁰B)boranethiol-cyclohexanone, ethyl E/Z-3-(undecahydrododeca(¹⁰B) boranethio)-acrylate, 3-(3-undecahydrododeca(¹⁰B)boranethio-propynoyl)-2-oxazolidinone and 2-[undecahydrododeca(¹⁰B)boranethiomethyl]-1-aza-bicyclo-[2.2.2]octan-3-one.

Such a compound can be suitably used as it is, or used in the form of a pharmaceutically acceptable salt, or used in the form of a pharmaceutical preparation known to a person with an ordinary skill in the art by mixing them with a pharmaceutically acceptable carrier, or used in the form of a BSH-enclosed viral envelope vector in a boron neutron capture therapy (BNCT). Examples of the pharmaceutically acceptable salt include salts with an inorganic base, salts with an organic base, salts with an inorganic acid, salts with an organic acid, salts with a basic or acidic amino acid and the like. Preferred examples of the salts with an inorganic base include alkali metal salts such as a sodium salt and a potassium salt; alkali earth metal salts such as a calcium salt and a magnesium salt; an aluminum salt, an ammonium salt and the like. Preferred examples of the salts with an organic base include salts with trimethylamine, triethylamine, pyridine, picoline, ethanolamine, diethanolamine, triethanolamine, dicyclohexylamine, N,N'-dibenzylethylenediamine and the like. Preferred examples of the salts with an inorganic acid include salts with hydrochloric acid, hydrobromic acid, nitric acid, sulfuric acid, phosphoric acid and the like. Preferred examples of the salts with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like. Preferred examples of the salts with a basic amino acid include salts with arginine, lysin, ornithine and the like. Preferred examples of the salts with an acidic amino acid include salts with aspartic acid, glutamic acid and the like.

The treatment is performed via any appropriate route of administration by administrating a drug containing the compound of the present invention using a method in which the compound is accumulated at the target site. The compound of the present invention is preferably concentrated to tumor. The pharmaceutical preparation containing the compound can be administered at a time, or can be sequentially administered. Administration of the pharmaceutical preparation can be optionally repeated. If desired, after removing the tumor to a surgically possible extent, the remaining tumor can also be destroyed using the pharmaceutical preparation of the present invention.

The treatment using the BSH derivative pharmaceutical preparation of the present invention is performed via any appropriate route of administration by administering using a method in which a BSH derivative is accumulated in the target tumor. The BSH derivative is preferably concentrated to the tumor before irradiation with radiation. A tumor/blood ratio before irradiation with radiation is advantageously about 2:1 or at least 1.5:1. The BSH derivative can be administered at a time, or can be sequentially administered. After the compound is desirably accumulated in the tumor, the site is irradiated with an effective amount of low energy neutron. The site can be irradiated through the skin, or the site can be completely or partially exposed before irradiation. Administration of the BSH derivative and the subsequent irradiation with radiation can be optionally repeated. If desired, after removing the tumor to a surgically possible extent, the remaining tumor is destroyed using the BSH derivative of the present invention. In another aspect, a proper amount of the BSH derivative may be administered to patients, followed by irradiation with an effective amount of ²⁵²Californium which is a naturally occurring neutron radiation substance. It is preferred that ²⁵²Californium is inserted into the tumor and then removed within a proper time.

In order to administer the BSH derivative of the present invention, the BSH derivative can be administered to patients by mixing with proper excipients, adjuvants and/or pharmaceutically acceptable carriers, alone or using in combination with other drugs. The carriers which can be particularly preferably used include, but are not limited to, physiological saline, buffered physiological saline, dextrose, water and the like. In an embodiment of the present invention, the pharmaceutically acceptable carriers are pharmaceutically inactive.

The BSH derivative of the present invention is administered orally and parenterally. In the case of parenteral administration, the BSH derivative can be administered intraarterially (for example, via carotid artery), intramuscularly, subcutaneously, intramedullary, intrathecally, intraventricularly, intravenously, intraperitoneally, or intranasally.

The pharmaceutical preparation can be formulated into any form such as powders, granules, fine grain agents, dry syrups, tablets, capsules, injections and liquids. According to the dosage form, using a pharmaceutically known technique, the pharmaceutical preparation can be prepared by appropriately mixing with, or diluting and dissolving together with pharmaceutical additives, for example, proper excipients; disintegrants; binders; lubricants; diluents; buffers such as organic acids including phosphoric acid, citric acid, succinic acid, acetic acid and other organic acids, or salts thereof; isotonizing agents; antiseptics; humectants; emulsifiers; dispersing agents; stabilizers; solubilizers; antioxidants such as ascorbic acid; low molecular (less than about 10 residues) polypeptides (for example, polyarginine or tripeptides); proteins (for example, serum albumin, gelatin, or immunoglobulin); hydrophilic polymers (for example, polyvinyl pyrrolidone); amino acids (for example, glycine, glutamic acid, aspartic acid, or arginine); monosaccharides, disaccharides and other carbohydrates (including cellulose or derivatives thereof, glucose, mannose, or dextrin); chelating agents (for example, EDTA); sugar alcohols (for example, mannitol or sorbitol); counter ions (for example, sodium); and/or nonionic surfactants (for example, polysolvate, poloxamer). Such a substance, which enhances isotonicity and chemical stability, is nontoxic to the recipient in the dose and concentration used.

Technologies for formulation and administration are described, for example, in the latest edition and the latest supplement of Japanese pharmacopoeia, and the final edition of "REMINGTON'S PHARMACEUTICAL SCIENCES" (Maack Publishing Co., Easton, PA).

A pharmaceutical preparation of the BSH derivative of the present invention is a drug in which the objective drug is contained in an effective amount for achieving the object, and a "therapeutically effective amount" or a "pharmaceutically effective amount" refers to the amount of the drug, which is sufficiently recognized by a person with in ordinary skill in the art and is effective to exert the pharmaceutical effect. Determination of a therapeutically effective dose is sufficiently known to a person with an ordinary skill in the art.

A therapeutically effective amount refers to the amount of the drug that alleviates the condition of a disease by administration. Therapeutic efficacy and toxicity of such compounds can be determined by standard pharmaceutical procedures in cell cultures or experimental animals. The dose is preferably within a range of the circulating concentration including ED50 with little or no toxicity. This dose may vary within this range depending upon the dosage form used, sensitivity of patients, and the route of administration. As an example, the dose of the composite is appropriately selected according to ages and other conditions of patients, kinds of diseases, and kind of composites used and the like.

Specific examples of the production method of a BSH derivative of the present invention will be described by way of aspects of examples, but the present invention is not limited thereto.

### Examples

In the following examples, analysis and isolation and purification of a compound were performed using the following models and reagents.
NMR spectrum: JEOL JMTC-400/54/SS 400 MHz (manufactured by JEOL, Ltd.). Unless otherwise specified, TMS was used as an internal standard. The following chemical shift was expressed by the δ value.
Silica gel for column chromatography: BW-200 (manufactured by FUJI SILYSIA CHEMICAL LTD.)

### (Example 1)

### Preparation of 3-[undecahydrododeca(¹⁰B)boranethiomethyl]-dihydrofuran-2-one

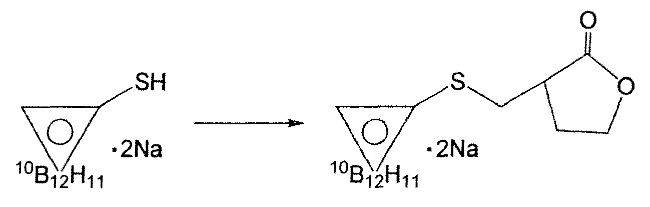

BSH·2Na (10 mg, 0.043 mmol) was dissolved in MeCN (1 mL), and NaOEt (2.0 wt% ethanol solution, 225 mL, 0.065 mmol) and a-methylene-g-butyrolactone (49 mg, 0.57 mmol) were added. After stirring for 3 hours, a solvent was concentrated, and then the residue was washed with Et₂O and dissolved in water (20 mL). This reaction mixture was desalinated by cation exchange chromatography (Amberlite IR-120, 2.5 x 4.0 cm, H₂O) and purified using HP-20 (1.5 x 10 cm, H₂O). The obtained fraction was collected and then neutralized by adding 0.1M NaOH (0.76 mmol)) to obtain an objective compound 2 (colorless oily product, yielded amount: 12 mg, yield: 93%).
¹H NMR (400MHz, D₂O) : 0.68-1.75 (11H, m, ¹⁰B₁₂H₁₁), 2.04-2.11 (1H, m, b-CH₂(each), 2.22-2.34 (1H, m, b-CH₂(each)), 2.49-2.55 (0.5H, m, a-CH₂(each)), 2.73-2.76(0.5H, m, a-CH₂(each)), 2.88-2.96 (1H, m, -SCH₂(each)), 3.39-3.46 (1H, m, -SCH₂(each)), 4.11-4.29 (2H, m, g-CH₂)

### (Example 2)

### Preparation of methyl 3-[undcahydrododeca(¹⁰B)boranethio]-propionate

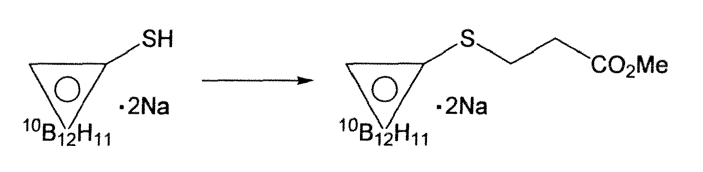

BSH·2Na (80 mg, 0.38 mmol) was dissolved in MeCN (2 mL), and NaOEt (39 mg, 0.57 mmol) and methyl acrylate (49 mg, 0.57 mmol) were added. After stirring for 1 hour, a solvent was concentrated, and then the residue was washed with Et₂O and dissolved in water (20 mL). This reaction mixture was desalinated by cation exchange chromatography (Amberlite IR-120, 2.5 x 4.0 cm, H₂O) and purified using HP-20 (1.5 x 10 cm, H₂O). The obtained fraction was collected and then neutralized by adding 0.1M NaOH (0.76 mmol) to obtain an objective compound (colorless oily product, yielded amount: 107 mg, yield: 100%).
¹H NMR(400MHz, D₂O) : 0.75-1.75 (11H, m, ¹⁰B₁₂H₁₁), 2.29 (2H, t, J=6.8Hz, a-CH₂), 3.19 (3H, s, COUCH₃), 3.55 (2H, t, J=6.8Hz, b-CH₂)

### (Example 3)

### Preparation of 3-methyl-4-[undecahydrododeca (¹⁰B) boranethio]-butan-2-one

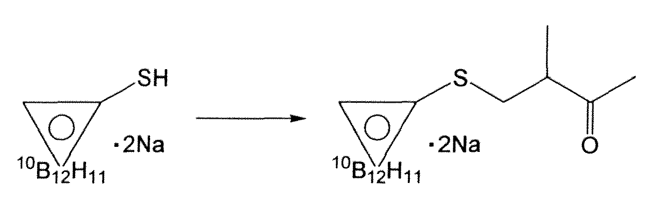

BSH·2Na (20 mg, 0.095 mmol) was dissolved in MeCN (2 mL), and dimethylaminopyridine (11 mg, 0.095 mmol) and 3-methyl-3-buten-2-one (9.6 mg, 0.11 mmol) were added. After stirring for 24 hours, a solvent was concentrated, and then the residue was washed with Et₂O and dissolved in water (20 mL). This reaction mixture was purified using HP-20 (1.5 x 10 cm, H₂O) to obtain an objective compound 10 (colorless oily product, yielded amount: 27 mg, yield: 96%).
¹H NMR(400MHz, CD₃CN) : 0.30-1.10 (11H, m, ¹⁰B₁₂H₁₁), 0.48 (3H, d, J = 0.68 Hz , 3-CH₃), 1.62 (3H, s, 1-CH₃), 2.10-2.21 (2H, dd, J=12.4Hz, 0.68Hz, 4-CH₂), 2.33-2.33 (1H, dt, J = 0.68 Hz, 3-CH)

### (Example 4)

### Preparation of 3-[undecahydrododeca(¹⁰B)boranethio]-cyclohexanone

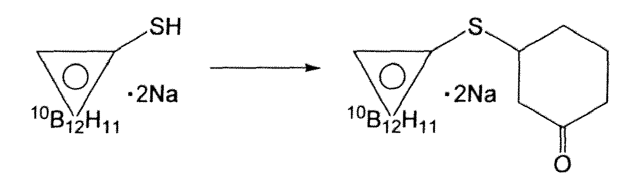

BSH·2Na (20 mg, 0.095 mmol) was dissolved in MeCN (2 mL), and dimethylaminopyridine (11 mg, 0.095 mmol) and cyclohexenone (11 mg, 0.11 mmol) were added. After stirring for 24 hours, a solvent was concentrated, and then the residue was washed with Et₂O and dissolved in water (20 mL). This reaction mixture was purified using HP-20 (1.5 x 10 cm, H₂O) to obtain an objective compound (colorless oily product, yielded amount: 26 mg, yield: 93%).
¹H NMR (400MHz, CD₃CN): 0.68-1.48 (11H, m, ¹⁰B₁₂H₁₁), 1.51-1.64 (2H, m, 5-CH₂), 1.95-2.03 (2H, m, 4-CH₂), 2.10-2.21 (2H, m, 6-CH₂), 2.23-2.29 (1H, m, 2-CH₂{each)), 2.62-2.67 (1H, m, 2-CH₂(each)), 2.95-3.04 (1H, m, 3-CH)

### (Example 5)

### Preparation of N^{α}- (p-cyanobenzoyl) -DL-3-(undecahydrododeca(¹⁰B)boranethio)-alanine methyl ester

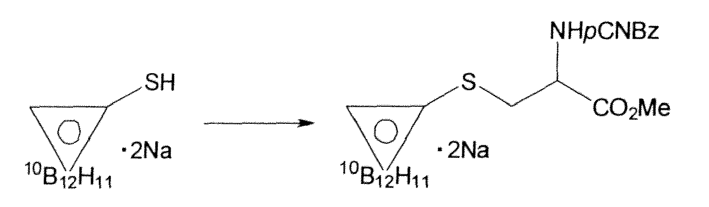

BSH·2Na (200 mg, 0.951 mmol)) was dissolved in MeCN(5 mL), and methyl N^{α}-(p-cyanobenzoyl)-2-aminoacrylate (227 mg, 1.05 mmol)) and dimethylaminopyridine (49 mg, 0.57 mmol) were added. After stirring for 24 hours, a solvent was concentrated, and then the residue was washed with Et₂O and dissolved in water (20 mL). This reaction mixture was purified using HP-20 (2.0 x 20 cm, H₂O) to obtain an objective compound (colorless oily product, yielded amount: 324 mg, yield: 86%).
¹H NMR (400MHz, D₂O) : 0.75-1.68 (11H, m, ¹⁰B₁₂H₁₁), 2.88-3.06 (2H, m, β-CH₂), 3.65 (3H, s, COUCH₃), 7.75 (2H, d, J=6.4Hz, 3-Ar-H), 7.84(2H, d, J=6.4Hz, 2-Ar-H)

### (Example 6)

### Preparation of ethyl E/Z-3-(undecahydrododeca (¹⁰B) boranethio)-acrylate (compound 15)

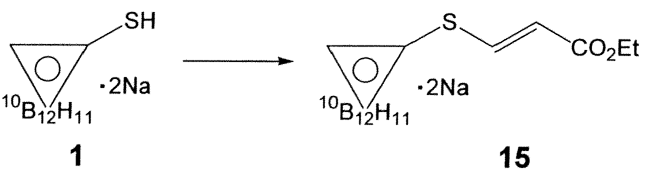

BSH·2Na (200 mg, 0.951 mmol) was dissolved in MeCN (5 mL), and ethyl propionate (103 mg, 1.05 mmol) and dimethylaminopyridine (49 mg, 0.57 mmol)) were added. After stirring for 24 hours, a solvent was concentrated, and then the residue was washed with Et₂O and dissolved in water (20 mL). This reaction mixture was purified using Hip-20 (2.0 x 20 cm, H₂O) to obtain an objective compound 15 as an E/Z mixture (colorless oily product, yielded amount: 290 mg, yield: 96%, E/Z = 6/1).
1H NMR (400MHz, D20): 0.75-1.68 (11H, m, 10B12H11), 1.14 (3H, t, J=7.2Hz, CH3), 4.04 (2H, q, J=7.2Hz, CH2), 5.70 (0.77H, d, J=10Hz, 2-CH), 5.86 (0.12H, t, J=15.6Hz, 2-CH2), 7.60 (0.71H, d, J=10Hz, 3-CH), 8.01 (0.11H, t, J=15.6Hz, 3-CH)

### (Example 7)

### Preparation of 3-(3-undecahydrododeca(¹⁰B)boranethio-propynoyl)-2-oxazolidinone (compound 16)

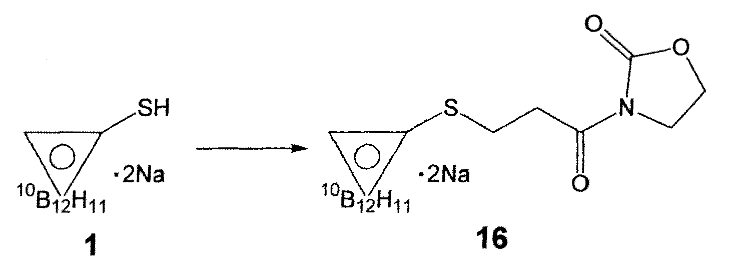

BSH·2Na (200 mg, 0.951 mmol) was dissolved in DMF (5 mL), and 3-(2-propenoyl)-2-oxazolidinone (148 mg, 1.05 mmol) and triethylamine (106 mg, 1.05 mmol) were added. After stirring for 48 hours, a solvent was concentrated, and then the residue was washed with Et₂O and dissolved in water (20 mL). This reaction mixture was purified using HP-20 (2.0 x 20 cm, H₂O) to obtain an objective compound 16 (colorless oily product, yielded amount: 275 mg, yield: 80%).
1H NMR(400MHz, D20): 0.60-1.80 (11H, m, 10B12H11), 2.59-2.69 (2H, br, -COCH2), 3.04-3.10 (2H, br, -SCH2), 3.91 (2H, t, J=8.0Hz, NCH2CH2O), 4.38 (2H, t, J=8.0Hz, NCH2CH2O)

In the same manner as in Examples 1 to 7, the following compounds were obtained.

**Table 1**

| | Compounds | (NMR value |
|---|---|---|
| 1 | 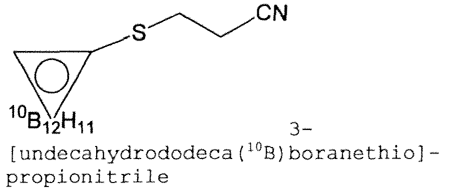 | ¹H NMR(400MHz, CD₃CN): 0.68-1.70 (11H, m, ¹⁰B₁₂H₁₁), 2.56 (2H, t, *J*=6. 8Hz, CH₂CN), 2.66 (2H, t, *J*=6.8Hz, SCH₂) |
| 2 | 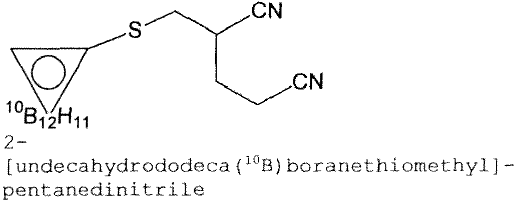 | ¹H NMR(400MHz, CDₛCN) : 0.65-1.60 (13H, m, ¹⁰B₁₂H₁₁ and NCCH₂CH₂CHCN), 2.88-2.98 (3H, m, NCCH₂ and CHCN), 3.01-3.04 (2H, m, SCH₂) |
| 3 | 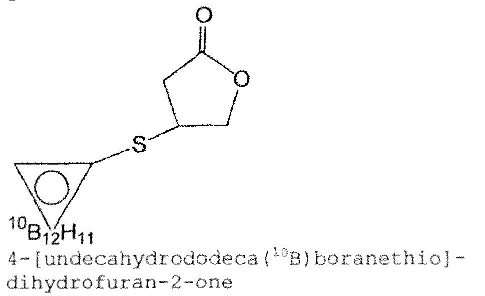 | ¹H NMR(400MHz, CD₃CN): 0.65-1.66 (11H, m, ¹⁰B₁₂H₁₁ 2.46-2.52 (2H, m, α-CH₂), 3.39-3.51 (1H, m, SCH), 4.42-4.56 (2H, m, γ-CH₂),* |
| 4 | 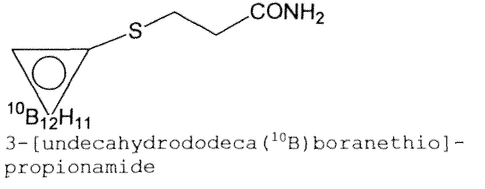 | ¹H NMR (400MHz, D₂O): 0.62-1.81 (11H, m, ¹⁰B₁₂H₁₁), to 2.41 (2H, m, CH₂CONH₂), 3.62 (2H, m, SCH₂) |

**Table 2**

| | | |
|---|---|---|
| 5 | 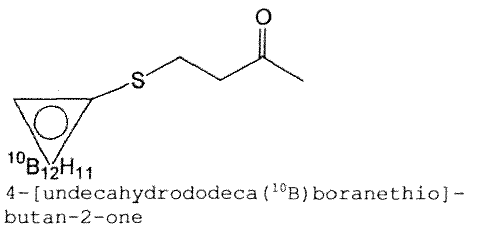 | ¹H NMR(400MHz, CD₃CN): 0.62-1.55 (11H, m, ¹⁰B₁₂H₁₁), 2.01 (3H, S, COCH₃), 2.52 (2H, t, *J*=7.6Hz, SCH₂), 2.66 (2H, t, *J*=7.6Hz, CH₂CO) |
| 6 | 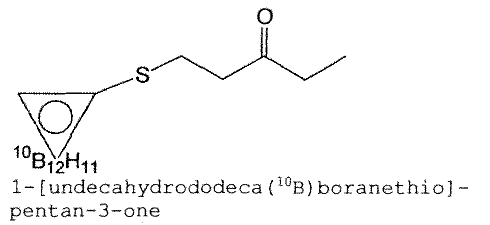 | ¹H NMR(400MHz, CD₃CN): 0.62-1.54 (14H, m, ¹⁰B₁₂H₁₁, and CH₃), 2.81 (2H, q, *J*=7.2Hz, COCH₂), 2.52 (2H, t, *J*=7.6Hz, SCH₂). 2.64 (2H, t, *J* 7.6he, CH₂C0) |
| 7 | 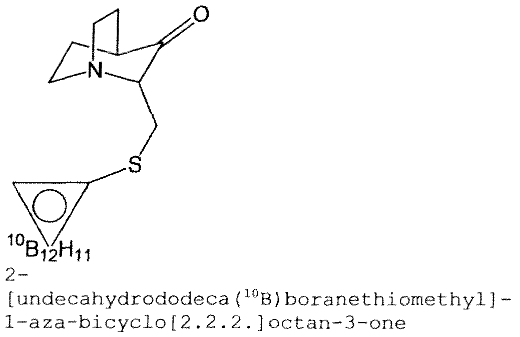 | ¹H NMR(400MHz, D₂O): 0.70-1.72 (15H, m, ¹⁰B₁₂H₁₁ and 3-CH₂), 2.11-2.38 (4H, m, 2-CH₂), 2.50-2.62 (2H, m, SCH₂), 3.81-3.88 (1H, m, CHCO) |
| 8 | 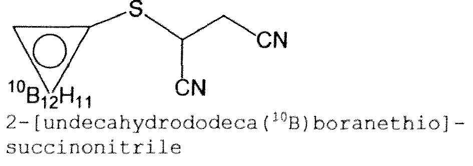 | ¹H NNR(400MHz, D₂O) : 0.73-1.75 (11H, m, ¹⁰B₁₂H₁₁), 2.93-3.07 (2H, m, SCHCN), 3.87 (1H, br, CH₂CN), |

**Table 3**

| | | |
|---|---|---|
| 9 | 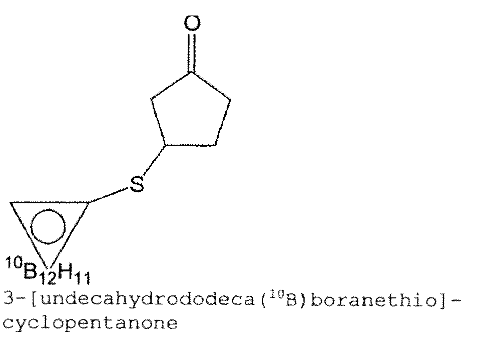 | ¹H NMR(400MHz, CD₃CN): 0.65.1.66 (11H, M, ¹⁰B₁₂H₁₁), 1.88-1.92 (2H, m, 5-CH₂), 1.99-2.20 (2H, m, 4-CH₂), 2.23-2.30 (2H, m, 1-CH₂), 3.40 (1H, br, SCH) |

## Claims

1. A method for producing a BSH derivative, comprising the step of an addition reaction of BSH with an α,β-unsaturated carbonyl compound or an α,β-unsaturated nitrile compound in the presence of a base.

2. The method for producing a BSH derivative according to claim 1, wherein the α,β-unsaturated carbonyl compound or the α,β-unsaturated nitrile compound is one kind selected from the group consisting of methyl N^{α}-(P-cyanobenzoyl)-2-aminoacrylate, acrolein, crotonaldehyde, cinnamaldehyde, mesityl oxide, benzalacetone, dibenzalacetone, benzalacetophenone, dypnone, acrylic acid, crotonic acid, isocrotonic acid, methacrylic acid, sorbic acid, cinnamic acid, maleic acid, fumaric acid, maleic anhydride, acrylonitrile, 2-butenedinitrile, methylene butyrolactone, oxofuran, methyl acrylate, acrylamide, methylene pentanedinitrile, methyl methacrylate, ethyl cinnamate, acrylonitrile, methyl vinyl ketone, cyclopentenone, methylcyclopentenone, cyclohexenone, propenoyloxazolidinone, ethyl propionate, methyl propionate, 3-methyl-3-buten-2-one, 1-penten-3-one and 2-methylenequinuclidin-3-one.

3. A BSH derivative represented by the general formula: 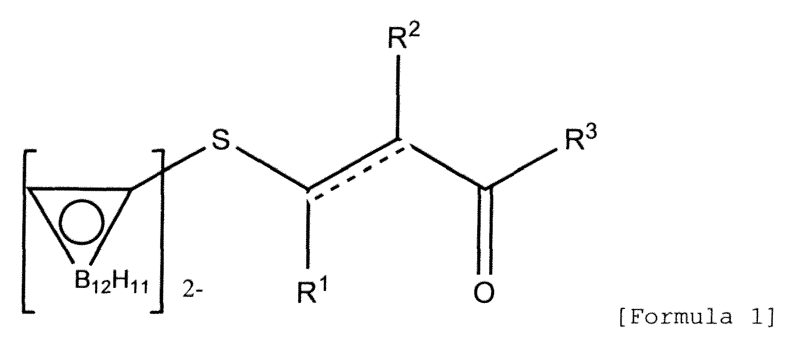 wherein a dashed line represents a bond or no bond; either R¹ or R² necessarily represents hydrogen, and both R¹ and R² may be hydrogens or either R¹ or R² may be an alkyl group having 1 to 6 carbon atoms, or a substituted amino group (in which a substituent represents a C₁₋₆ alkyl group or a cyanobenzoyl group); and R³ represents hydrogen, an alkyl group having 1 to 6 carbon atoms, an alkoxyl group having 1 to 6 carbon atoms (provided that, when R³ is a C1 alkoxyl group and a dashed line is no bond, R² is not hydrogen) or NR⁴R⁵ (in which R⁴ and R⁵ independently represent hydrogen, an alkyl group having 1 to 6 carbon atoms, or NR⁴R⁵s are combined together to form a 4- to 10-membered, saturated or unsaturated ring having a hetero atom); or when R¹ is hydrogen, R² and R³ represent, together with adjacent carbonyl groups, an oxo-substituted C4-10 cycloalkane, an oxo-substituted unsaturated hydrocarbon ring group having 4 to 10 carbon atoms, a 4- to 10-membered oxy-substituted saturated or unsaturated heterocyclic group having at least one oxygen or nitrogen or sulfur atom, an oxo-substituted C6-12 crosslinked cyclic hydrocarbon group, or a 6- to 12-membered oxo-substituted crosslinked ring group having at least one oxygen or nitrogen or sulfur atom), or a pharmaceutically acceptable salt thereof.

4. A BSH derivative represented by the general formula: 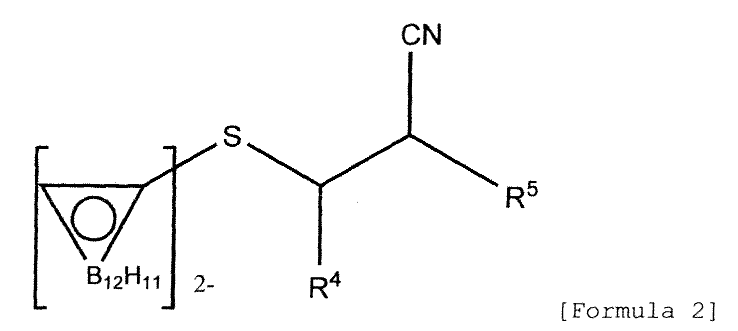 wherein either R⁴ or R⁵ necessarily represents hydrogen, but both R⁴ and R⁵ are not hydrogens, and either R⁴ or R⁵ represents an alkyl group having 1 to 6 carbon atoms, a cyano C1-6 alkyl group or cyano, or R⁴ and R⁵ represent, together with adjacent cyano groups, a cyano group-substituted C4-10 cycloalkane, a cyano-substituted unsaturated hydrocarbon ring group having 4 to 10 carbon atoms, a 4- to 10-membered cyano-substituted saturated or unsaturated heterocyclic group having at least one oxygen or nitrogen or sulfur atom, a cyano-substituted C6-12 crosslinked cyclic hydrocarbon group, or a 6- to 12-membered cyano-substituted crosslinked ring group having at least one oxygen or nitrogen or sulfur atom), or a pharmaceutically acceptable salt thereof.

5. A BSH derivative which is one kind selected from the group consisting of N^{α}-(p-cyanobenzoyl)-DL-3-(undecahydrododeca (¹⁰B) boranethio)-alanine methyl ester, 3-[undecahydrododeca (¹⁰B) boranethiomethyl]-dihydrofuran-2-one, 2-[undecahydrododeca (¹⁰B) boranethiomethyl]pentanedinitrile, 4- [undecahydrododeca (¹⁰B) boranethio]-dihydrofuran-2-one, methyl 3-[undecahydrododeca(¹⁰B)boranethio]-propionate, 2-[undecahydrododeca(¹⁰B)boranethio]-succinonitrile, 3-[undecahydrododeca(¹⁰B)boranethio]propionamide, 4-[undecahydrododeca(¹⁰B)boranethio]-butan-2-one, 3-[undecahydrododeca(¹⁰B)boranethio]-cyclopentanone, 3-methyl-4-[undecahydrododeca(¹⁰B)boranethio]-butan-2-one, 1-[undecahydrododeca (¹⁰B) boranethio]-pentan-3-one, 3-[undecahydrododeca(¹⁰B)boranethio]-cyclohexanone, ethyl E/Z-3-(undecahydrododeca (¹⁰B)boranethio)-acrylate, 3-(3-undecahydrododeca (¹⁰B) boranethio-propynoyl)-2-oxazolidinonedinone and 2-[undecahydrododeca (¹⁰B) boranethiomethy]-1-aza-bicyclo-[2.2.2]octan-3-one, or a pharmaceutically acceptable salt thereof.

## Amended claims

### Amended claims under Art. 19.1 PCT

**1.** A method for producing a BSH derivative, comprising the step of an addition reaction of BSH with an α,β-unsaturated carbonyl compound or an α,β-unsaturated nitrile compound in the presence of a base.

**2.** The method for producing a BSH derivative according to claim 1, wherein the α,β-unsaturated carbonyl compound or the α,β-unsaturated nitrile compound is kind selected from the group consisting of methyl N^{α}-(P-cyanobenzoyl)-2-aminoacrylate, acrolein, crotonaldehyde, cinnamaldehyde, mesityl oxide, benzalacetone, dibenzalacetone, benzalacetophenone, dypnone, acrylic acid, crotonic acid, isocrotonic acid, methacrylic acid, sorbic acid, cinnamic acid, maleic acid, fumaric acid, maleic anhydride, acrylonitrile, 2-butenedinitrile, methylene butyrolactone, oxofuran, methyl acrylate, acrylamide, methylene pentanedinitrile, methyl methacrylate, ethyl cinnamate, acrylonitrile, methyl vinyl ketone, cyclopentenone, methylcyclopentenone, cyclohexenone, propenoyloxazolidinone, ethyl propionate, methyl propionate, 3-methyl-3-buten-2-one, 1-penten-3-one and 2-methylenequinuclidin-3-one.

**3.** A BSH derivative represented by the general formula: 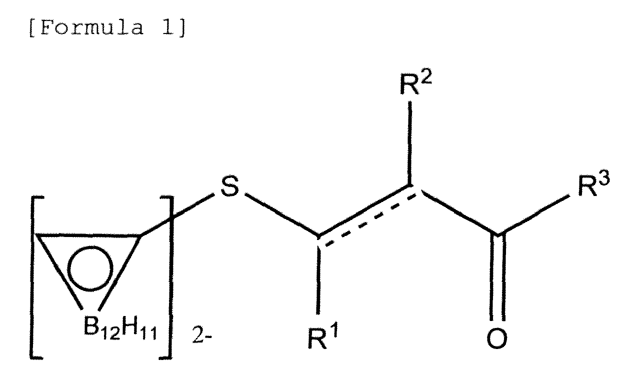 wherein a dashed line represents a bond or a no bond; either R¹ or R² necessarily represents hydrogen, and both R¹ and R² may be hydrogens or either R¹ or R² may be an alkyl group having 1 to 6 carbon atoms, or a substituted amino group (in which a substituent represents a C1-6 alkyl group or a cyanobenzoyl group); and R³ represents hydrogen, an alkyl group having 1 to 6 carbon atoms, an alkoxyl group having 1 to 6 carbon atoms (provided that, when R³ is a C1 alkoxyl group, and a dashed line is no bond, R² is not hydrogen) or NR⁴R⁵ (in which R⁴ and R⁵ independently represent hydrogen, an alkyl group having 1 to 6 carbon atoms, or NR⁴R⁵s are combined together to form a 4- to 10-membered, saturated or unsaturated ring having a hetero atom); or when R¹ is hydrogen, R² and R³ represent, together with adjacent carbonyl groups, an oxo-substituted C4-10 cycloalkane, an oxo-substituted unsaturated hydrocarbon ring group having 4 to 10 carbon atoms, a 4- to 10-membered oxo-substituted saturated or unsaturated heterocyclic group having at least one oxygen or nitrogen or sulfur atom, an oxo-substituted C6-12 crosslinked cyclic hydrocarbon group, or a 6- to 12-membered oxo-substituted crosslinked ring group having at least one oxygen or nitrogen or sulfur atom), or a pharmaceutically acceptable salt thereof.

**4.** A BSH derivative represented by the general formula: 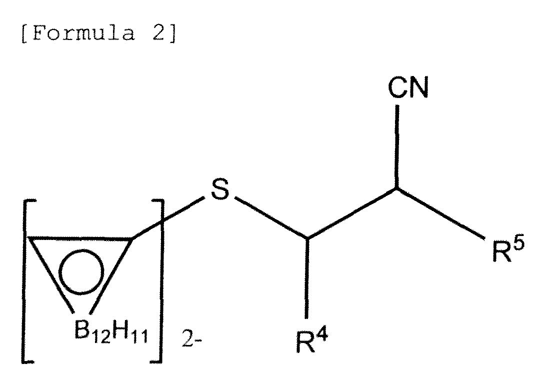 wherein either R⁴ or R⁵ necessarily represents hydrogen, but both R⁴ and R⁵ are not hydrogens, and either R⁴ or R⁵ represents an alkyl group having 1 to 6 carbon atoms, a cyano C1-6 alkyl group or cyano, or R⁴ and R⁵ represent, together with adjacent cyano groups, a cyano group-substituted C4-10 cycloalkane, a cyano-substituted unsaturated hydrocarbon ring group having 4 to 10 carbon atoms, a 4- to 10-membered cyano-substituted saturated or unsaturated heterocyclic group having at least one oxygen or nitrogen or sulfur atom, a cyano-substituted C6-12 crosslinked cyclic hydrocarbon group, or a 6- to 12-membered cyano-substituted crosslinked ring group having at least one oxygen or nitrogen or sulfur atom), or a pharmaceutically acceptable salt thereof.

**5.** (After amendment)
A BSH derivative which is one kind selected from the group consisting of N^{α}-(p-cyanobenzoyl)-DL-3-(undecahydrododeca(¹⁰B)boranethio)-alanine methyl ester, 3-[undecahydrododeca(¹⁰B)boranetyhiomethyl-dihydrofuran-2-one, 2-[undecahydrododeca(¹⁰B)boranethiomethyl]pentanedinztrile, 4-[undecahydrododeca(¹⁰B)boranethio]-dihydrofuran-2-one, 2-[undecahydrododeca(¹⁰B) boranethio]-succinonitrile, 3-[undecahydrododeca(¹⁰B)boranothio]propionamide, 4-[undecahydrododeca(¹⁰B)boranethio]-butan-2-one, 3-[undecahydrododeca(¹⁰B)boranethio]-cyclopentanone, 3-methyl-4-[undecahydrododeca(¹⁰B)boranethiol-butan-2-one, 1-[undecahydrododeca(¹⁰B) boranethio]-pentan-3-one, 3-[undecahydrododeca (¹⁰B) boranethio]-cyclohexanone, ethyl E/Z-3-(undecahydrododeca(¹⁰B)boranethio)-acrylate, 3-(3-undecahydrododeca (¹⁰B)boranethio-propynoyl)-2-oxazolidinone, and 2-[undecahydrododeca (¹⁰B)boranethiomethyl]-1-aza-bicyclo-[2.2.2]octan-3-one, or a pharmaceutically acceptable salt thereof.
